# EUROPEAN PATENT APPLICATION

(11) **EP 2 368 883 A1**
(43) Date of publication of application: **28.09.2011**
(21) Application number: 10003126.9
(22) Date of filing: 24.03.2010
(51) Int. Cl.: C07D 239/42

(54) **Process for the preparation of 2-substituted 4-amino-5-cyanopyrimidines**

(71) Applicant: Lonza Ltd, 4052 Basel (CH)
(72) Inventor: Hanselmann, Paul, 3902 Brig-Glis (CH); Dick, Victor, 4054 Basel (CH); Ladnak, Victor, 3604 Thun (CH); Kramer, Rainer, 3931 Lalden (CH)

(57) **Abstract**

A compound of the formula 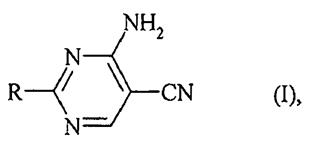 wherein R¹ is C₁₋₄ alkyl or phenyl, is prepared by reacting a dicyanomethanide of the formula 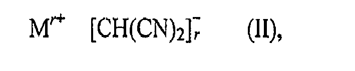 wherein M is an alkali or alkaline earth metal and *r* is 1 or 2, with carbon monoxide to obtain a dicyanoethenolate of the formula 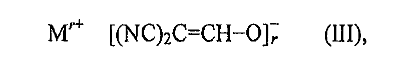 which is acylated to obtain an acyloxymethylenemalononitrile of the formula

(NC)₂=CH-OCOR² (IV),

wherein R² is C₁₋₄ alkyl, which in turn is reacted with an amidine of the formula 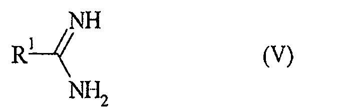 to obtain the 4-amino-5-cyanopyrimidine of the formula I.
The compound of formula I, wherein R is methyl, is an intermediate in a synthesis of thiamin (vitamin B₁).

## Description

The present invention relates to an improved process for the preparation of 2-substituted 4-amino-5-cyanopyrimidines.

4-Amino-5-cyano-2-methylpyrimidine is a useful intermediate for the preparation of thiamin (vitamin B₁) (R. Grewe, Hoppe-Seylers Z. Physiol. Chem. 1936, 242, 89-96). However, the prior art syntheses of 4-amino-5-cyano-2-methylpyrimidine do not fulfill all the desired criteria for the production on an industrial scale. Such crucial criteria are for example prices of starting materials, reagents and solvents; safety; working hygiene; ecology; energy consumption; number of reaction steps; reaction rate; temperature requirements; complexity of purification; or apparatus complexity.

The object of the present invention is therefore to find an improved process for the preparation of 4-amino-5-cyano-2-methylpyrimidine and other 2-substituted 4-amino-5-cyanopyrimidine.

The present invention relates to a process for the preparation of a compound of the formula 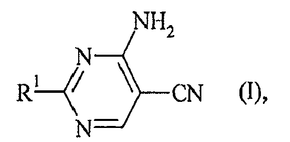 wherein R¹ is C₁₋₄alkyl or phenyl, which comprises the steps of
(i) reacting a dicyanomethanide of the formula 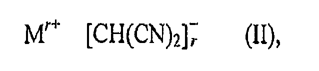 wherein M is an alkali metal or alkaline earth metal, and *r* is 1 or 2,
   with carbon monoxide to obtain a dicyanoethenolate of the formula 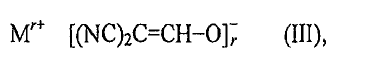 wherein M and *r* are as defined above,
(ii) reacting said dicyanoethenolate (III) with an acylating agent to obtain an acyloxy methylenemalononitrile of the formula

   (NC)₂C=CH-OCOR² (IV),

   wherein R² is C₁₋₄alkyl,
   and
(iii) reacting said acyloxymethylenemalononitrile (IV) with an amidine of the formula 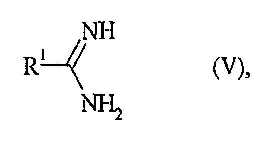 wherein R¹ is as defined above,
to obtain the 4-amino-5-cyanopyrimidine of the formula I.

In a preferred embodiment, the amidine (V) is acetamidine, R¹ in formulas I and V thus being methyl.

In another preferred embodiment, R² in formula IV is methyl and the acylating agent in step (ii) is selected from the group consisting of ketene, acetyl chloride, and acetic anhydride.

The dicyanomethanide (II) may, for example, be generated by reacting malononitrile and an alkoxide of the formula

M(OR³), (VI),

wherein M and *r* are as defined above and R³ is C₁₋₄ alkyl. In a preferred embodiment, this generation takes place *in situ.*

Suitable alkali metal alkoxides are for example lithium methoxide, lithium ethoxide, lithium isopropoxide, lithium butoxide, sodium methoxide, sodium ethoxide, sodium isopropoxide, sodium butoxide, potassium methoxide, potassium ethoxide, potassium isopropoxide or potassium butoxide. Of special interest are sodium methoxide and sodium ethoxide.

Suitable alkaline earth metal alkoxides are for example magnesium methoxide, magnesium ethoxide, magnesium isopropoxide, magnesium butoxide, calcium methoxide, calcium ethoxide, calcium isopropoxide, calcium butoxide, barium methoxide, barium ethoxide, barium isopropoxide or barium butoxide. Of special interest are calcium methoxide and calcium ethoxide.

Particularly preferred alkoxides (VI) are the methoxides, i.e. those where R³ is methyl.

Also particularly preferred are the sodium alkoxides, i.e., the alkoxides (VI) wherein M is Na and *r* is 1.

In another preferred embodiment the acyloxymethylenemalononitrile (IV) obtained in step (ii) is not isolated before reacting it with the amidine (V).

In another preferred embodiment the amidine (V) is generated *in situ* from a suitable salt, in particular the hydrochloride, and a strong base.

More preferably, the strong base is an alkoxide of the formula

M(OR³)*ᵣ* (VI),

wherein M is an alkali metal or alkaline earth metal, *r* is 1 or 2, and R³ is C₁₋₄ alkyl. Said alkoxide may be the same as or different from the alkoxide, if any, that is used to generate the dicyanomethanide (II) as described above.

Conveniently, the reaction of (II) with carbon monoxide (step (i)) takes place in a solvent. Suitable solvents include both polar and non-polar solvents, as well as mixtures thereof.

Polar solvents are for example alcohols, including diols and polyols, acyclic and cyclic ethers, and nitriles. Examples of alcohols are methanol, ethanol, propanol, isopropyl alcohol, butanol, pentanol, hexanol, ethylene glycol, diethylene glycol, glycerine and polyethylene glycol. Of special interest are methanol, ethanol and isopropyl alcohol.

Examples of cyclic ethers are 1,4-dioxane and tetrahydrofuran.

A typical example for a nitrile solvent is acetonitrile.

Non-polar solvents are for example aromatic hydrocarbons, such as benzene, toluene, o-xylene, *m*-xylene or *p*-xylene. Of very special interest is toluene.

Preferably, the reaction of the dicyanomethanide and carbon monoxide (step (i)) takes place in a polar solvent as defined above. Particularly suitable polar solvents comprise at least one alcohol, preferably a C₁₋₄ alcohol and most preferably the alcohol that forms the alkoxide moiety in the alkali or alkaline earth metal alkoxide used to generate the dicyanomethanide (II) and/or the amidine (V), or mixtures of said alcohols with one or more other polar or non-polar solvent(s).

A particularly preferred alcohol is methanol, in particular if the alkoxide used to prepare the dicyanomethanide as mentioned above is an alkali or alkaline earth metal methoxide. Methanol can be used as such, or in a mixture of methanol and toluene. The preferred molar ratio between methanol and toluene in said mixtures is between 10:1 and 1:10.

A preferred reaction temperature range for the reaction of the dicyanomethanide (II) and carbon monoxide is from 10 to 80 °C, preferably from 20 to 70 °C, for example 20 to 50 °C. An especially preferred reaction temperature range is 25 to 35 °C, for example 29 to 31 °C.

Preferably, the carbon monoxide pressure in the reaction of malononitrile and carbon monoxide is in the range of 20 to 70 bar, for example 40 to 70 bar. An especially preferred pressure range is from 50 to 60 bar.

The formation of the dicyanomethanide (II) and its reaction with carbon monoxide as well as the subsequent acylation and the formation of the cyanopyrimidine (I) from (IV) and (V) may also be conducted in a "one-pot" manner.

The instant invention relates also to the use of a compound of the formula I, wherein R is methyl, prepared according to the above process, for the preparation of thiamin (vitamin B₁).

The following examples illustrate the invention in more detail.

### Example 1

### Sodium 2,2-dicyanoethenolate (II, M = Na, r =1)

To a solution of sodium methoxide (27.8 g, 0.5 mol) in a mixture of methanol (142 mL) and toluene (82 mL) at 0 °C was added dropwise a solution of malononitrile (30.0 g, 0.45 mol) in methanol (6 mL) (exothermic reaction!). After the complete addition the reaction mixture was stirred for one hour at 0 °C. Then the reaction mixture was transferred into a high pressure autoclave. The autoclave was flushed with nitrogen (4 times at 3-5 bar) and afterwards with carbon monoxide (3 times at 5-10 bar). Afterwards the autoclave was charged with carbon monoxide (60 bar) and the temperature was adjusted to 30 °C. The reaction mixture was stirred for 16 hours while the carbon monoxide pressure was continuously controlled. The resulting suspension was filtered and the residue washed with toluene (3x30 mL). Drying under reduced pressure at 50 °C afforded the sodium 2,2-dicyanoethenolate as a white powder.

Yield: 41.8 g (78.6%)

### Example 2

### 4-Amino-5-cyano-2-methylpyrimidine (I, R = methyl)

To a solution of sodium 2,2-dicyanoethenolate (2.0 g, 16.9 mmol, prepared according to Example 1) in 1,4-dioxane (8 mL) was added acetamidine hydrochloride (1.98 g, 20.3 mmol) and acetic anhydride (1.91 mL). The reaction mixture was heated under reflux for 14 hours. Afterwards the solvent was completely evaporated and the resulting solid was taken up in hot water (80 °C). The suspension was cooled to room temperature under stirring, then filtered and washed with water. Drying of the residue under reduced pressure at 60 °C gave 4-amino-5-cyano-2-methylpyrimidine as a white powder.

### Example 3

### 4-Amino-5-cyano-2-methylpyrimidine (I, R = methyl)

### Reagent 1:

To a solution of sodium 2,2-dicyanoethenolate (7.6 g, 70 mmol, prepared according to Example 1) in dichloromethane (45.6 mL) was added acetyl chloride (5.3 g, 70 mmol) within 30 min. After complete addition the mixture was heated under reflux (about 43 °C) for 1.5 h, then cooled to about 0 °C and afterwards stirred for additional 1.5 h. The mixture was used as reagent 1.

### Reagent 2:

Acetamidine hydrochloride (18.42 g, 0.19 mol) was dissolved in ethanol (37.5 mL). Sodium methoxide (10.94 g, 0.2 mol) was added within 5 min and the reaction mixture was stirred for about 30 min. The resulting white suspension was filtered and the filter cake washed with ethanol. The filtrate comprising the acetamidine was used as reagent 2.

Within 45 min Reagent 2 was added dropwise to Reagent 1 at 0°C and stirred for additional 2.5 h. The resulting orange suspension (104 g) was concentrated by evaporating the solvent under reduced pressure (50 °C/650 mbar) to about ⅓ volume (28.8 g). Water (35 mL) was added to the residue to obtain an orange suspension which was filtrated with a P3 frit. The filter cake was washed four times with water (10, 20, 25, and 30 mL, wherein the first 10 mL where prior used to wash the vessel). Finally the filter cake was dried at 17 mbar/45 °C to obtain a yellow solid.

Yield: 3.65 g (42.2%, based on sodium 2,2-dicyanoethenolate)

Alternatively the product can be obtained by extraction with an organic solvent such as dichloromethane, diethyl ether, or ethyl acetate.

### Example 4

### Acetoxymethylenemalononitrile (IV, R² = methyl)

Acetyl chloride (8.3 g, 0.11 mol) was added dropwise within 30 min at room temperature to a stirred suspension of sodium 2,2-dicyanoethenolate (12.0 g, 0.1 mol) in dry dichloromethane (70 mL). The resulting mixture was stirred for another 3 h under reflux and the cooled to room temperature. The suspension was filtered to remove precipitated sodium chloride and the filter cake was washed with dichloromethane (3 x30 mL). The combined filtrates were concentrated under reduced pressure and the residue was distilled *in vacuo.*

Yield: 6.6 g (47%)

Bp.: 88-91 °C/0.5 Torr

¹H NMR (CDCl₃, 400 MHz): δ 2.42 (s, 3H), 8.48 (s, 1H).

### Example 5

### Sodium 2,2-dicyanoethenolate (II, M = Na, r =1)

In a high pressure autoclave, a solution of malononitrile (150.0 g, 2.27 mol) in methanol (30 mL) was added dropwise (1.2 g/min) at 0 °C to a solution of sodium methoxide (126.5 g @ 97%, 2.27 mol) in methanol (1.25 L). After the complete addition the reaction mixture was stirred for one hour at 0 °C and 30 min at room temperature. The autoclave was then flushed with nitrogen (4 times at 3-5 bar) and afterwards with carbon monoxide (3 times at 5-10 bar). Afterwards the autoclave was charged with carbon monoxide (30 bar) and the temperature was adjusted to 30 °C. The reaction mixture was stirred until the carbon monoxide consumption ceased (ca. 22 h). The resulting suspension was filtered and the filter cake washed with methanol (200 g) and toluene (190 g). The combined filtrates were concentrated *in vacuo* and toluene (160 g) was added to obtain a suspension that was filtered to obtain a second crop of product which was washed twice with toluene (200 g + 130 g). A third crop was obtained in a similar way from the combined filtrates of the second filtration. The solid product was dried *in vacuo* (40 °C, 20-50 mbar).

Yield: 206.4 g (78%).

## Claims

1. A process for the production of a 4-amino-5-cyanopyrimidine of the formula 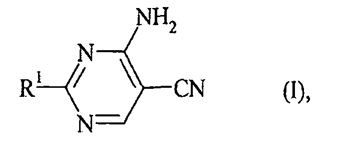 wherein R is C₁₋₄ alkyl or phenyl, which process comprises the steps of
(i) reacting a dicyanomethanide of the formula 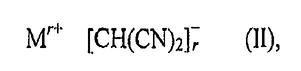 wherein M is an alkali metal or alkaline earth metal, and *r* is 1 or 2,
with carbon monoxide to obtain a dicyanoethenolate of the formula 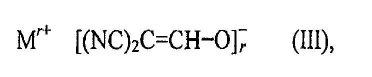 wherein M and *r* are as defined above,
(ii) reacting said dicyanoethenolate (III) with an acylating agent to obtain an acyloxymethylenemalononitrile of the formula
(NC)₂C=CH-OCOR² (IV),
wherein R² is C₁₋₄ alkyl, and
(iii) reacting said acyloxymethylenemalononitrile (IV) with an amidine of the formula 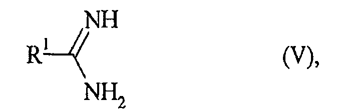 wherein R¹ is as defined above,
to obtain the 4-amino-5-cyanopyrimidine of the formula I.

2. The process of claim 1, wherein R¹ is methyl.

3. The process of claim 1 or 2, wherein R² is methyl and the acylating agent is selected from the group consisting of ketene, acetyl chloride and acetic anhydride.

4. The process of any of claims 1 to 3, wherein the dicyanomethanide (II) is prepared *in situ* from malononitrile and an alkoxide of the formula
M(OR³), (VI),
wherein M and *r* are as defined in claim 1 and R³ is C₁₋₄ alkyl.

5. The process of claim 4, wherein R³ is methyl.

6. The process of any of claims 1 to 5, wherein M is sodium and *r* is 1.

7. The process of any of claims 1 to 6, wherein the acyloxymethylenemalononitrile (IV) obtained in step (ii) is not isolated before being reacted with the amidine (V).

8. The process of any of claims 1 to 6, wherein the amidine (V) is generated *in situ* from the corresponding hydrochloride and a strong base.

9. The process of claim 8, wherein the strong base is an alkoxide of the formula
M(OR³)*ᵣ* (VI),
wherein M and *r* are as defined in claim 1 and R³ is C₁₋₄ alkyl.

10. The process of any of claims 1 to 9, wherein step (i) is conducted in a polar solvent.

11. The process of claim 10, wherein the polar solvent comprises at least one alcohol.

12. The process of claim 11, wherein the alcohol is methanol.

13. The process of any of claims 1 to 12, wherein step (i) is conducted at a temperature of 10 to 80 °C.

14. The process of any of claims 1 to 13, wherein step (i) is conducted at a carbon monoxide pressure of 20 to 70 bar.

15. The use of a compound of the formula I, wherein R is methyl, produced according to the process of claim 2, for the preparation of thiamin.
